# EUROPÄISCHE PATENTANMELDUNG

(11) **EP 1 217 573 A1**
(43) Veröffentlichungstag der Anmeldung: **26.06.2002**
(21) Anmeldenummer: 00811232.8
(22) Anmeldetag: 22.12.2000
(51) Int. Cl.: G06K 9/20, A61B 5/117

(54) **Vorrichtung zur Aufnahme der Papilarrillen eines Fingers**

(71) Anmelder: Fingerpin AG, 8053 Zürich (CH)
(72) Erfinder: Spycher, Martin, CH-8032 Zürich (CH); Golsong Roosdorp, Anne-Sophie, CH-6300 Zug (CH)
(74) Vertreter: Isler & Pedrazzini AG

(57) **Zusammenfassung**

Eine Vorrichtung zur optischen Aufnahme der Papilarrillen auf einer Fingeroberfläche weist einen transparenten Körper (1) mit einer Auflagefläche (2) auf. Licht von einer Lichtquelle (8) fällt auf die Auflagefläche (2) in Winkeln, die kleiner sind als der kritische Winkel für interne Totalreflexion an der Auflagefläche (2). Im Bereich der Berge (3) der Papilarrillen des Fingers werden evaneszente Felder angeregt, wobei propagierendes Licht (12,13,14) von der Auflagefläche (2) ausgeht und durch eine Abbildungsoptik (21) auf einen Lichtdetektor (22) fällt. Die Lichtstrahlen in dem Lichtbündel (12) gehen in Winkeln von der Auflagefläche (2) aus, die innerhalb des Winkelbereichs für intern totalreflektierte Lichtstrahlen liegt. Die Vorrichtung erbringt den Vorteil , dass der Kontrast einer Aufnahme von Papilarrillen unabhängig davon ist, ob die Fingeroberfläche nass oder trocken ist.

## Beschreibung

### Technisches Gebiet

Die Erfindung betrifft eine Vorrichtung zur optischen Aufnahme und Erstellung eines Kontrastbildes der Papilarrillen an der Oberfläche eines Fingers. Sie wird zum Beispiel in einem System für Zutrittskontrolle zur Identifizierung oder Verifizierung von Personen eingesetzt. Bei der optischen Aufnahme der Papilarrillen wird insbesondere die interne Totalreflexion von Lichtstrahlen an der Oberfläche des Fingers, welcher auf einen transparenten Körper gelegt wird, angewendet.

### Stand der Technik

Solche Geräte zur optischen Aufnahme eines Kontrastbildes der Papilarrillen eines Fingers weisen ein Prisma oder einen ähnlichen transparenten Körper auf, auf dessen Oberfläche der Finger gelegt wird. Die abzubildenden Papilarrillen des Fingers werden von einer Lichtquelle beleuchtet, die unterhalb des Prismas angeordnet ist und ihr Licht durch den transparenten Körper und auf die Papilarrillen wirft. Es ist bekannt, dass wenn Licht unter Einfallswinkeln grösser oder gleich einem kritischen Winkel auf eine Grenzfläche fällt zwischen einem dichteren Medium, wie zum Beispiel Glas, und einem weniger dichten Medium, wie zum Beispiel Luft, interne Totalreflexion erfährt und in das dichtere Medium zurückreflektiert wird. Der kritische Winkel beträgt ϑₖᵣᵢₜ = sin⁻¹(n₂/n₁), wobei n₂ der optische Brechungsindex des weniger dichten Mediums und n₁ der optische Brechungsindex des dichteren Mediums ist und ϑₖᵣᵢₜ der Winkel zwischen der Normalen zur Grenzfläche und dem einfallenden sowie auch dem total reflektierten Lichtstrahl ist. Befindet sich jedoch auf der Grenzfläche oder in der unmittelbaren Nähe der Grenzfläche ein Gegenstand, so kann das Licht aus dem Prisma austreten, was unter sogenannter verhinderter interner Totalreflexion bekannt ist. Dieses Phänomen wird zur optischen Aufnahme der Papilarrillen eines Fingers mittels interner Totalreflexion angewendet. Eine typische Anordnung ist zum Beispiel in Optics & Photonics News, October 2000, S.24-25 offenbart. Dort ist eine Lichtquelle auf eine der 45°-Flächen eines Prismas gerichtet, sodass ihr Licht durch das Prisma von unten auf die Hypotenuse des Prismas fällt, wobei der Einfallswinkel auf die Hypotenuse grösser ist als der kritische Winkel für interne Totalreflexion. Ein Finger liegt auf der Oberfläche der Hypotenuse, wobei der Finger jedoch nicht in gleichmässigem Kontakt mit dem Prisma ist. Von den Papilarrillen an der Fingerhaut sind nämlich nur die Berge der Rillen in Kontakt mit dem Prisma, und die Täler der Rillen sind eine kleine Distanz von der Prismaoberfläche entfernt. Das Licht erfährt in jenen Bereichen, wo die Berge der Rillen sich befinden, verhinderte interne Totalreflexion und tritt aus dem Prisma heraus. In den Tälern, wo sich Luft in einem Bereich grösser als die sogenannte evaneszente Abklingdistanz (welche typischerweise ca. 1 Mikrometer beträgt) befindet, wird das Licht total reflektiert und gelangt durch das Prisma und eine Abbildungsoptik zu einer Abbildungsfläche. Dort entsteht ein Kontrastbild der Papilarrillen des Fingers, bei dem die Berge dunkel und die Täler hell erscheinen.

Eine solche Anordnung zur Aufnahme der Papilarrillen eines Fingers ermöglicht ein kontrastreiches Bild der Papilarrillen sofern der Finger trocken ist und in den Tälern der Rillen sich Luft befindet. Ist jedoch der Finger nass oder ölig, was in der Praxis zum Beispiel an warmen Tagen oft vorkommt, so ist die Bedingung für interne Totalreflexion verändert, da sich nun statt Luft Wasser oder Öl auf der Prismaoberfläche und insbesondere in den Tälern der Rillen befindet. Der kritische Winkel für interne Totalreflexion ist dann aufgrund des höheren Brechungsindexes von Wasser oder Öl grösser als im Fall von Luft auf der Prismaoberfläche. In diesem Fall erfährt das Licht auch in den Bereichen der Täler verhinderte interne Totalreflexion und tritt aus dem Prisma und in das Wasser oder Öl. Es wird also nicht mehr reflektiert. Der Kontrast der Abbildung geht dadurch weitgehend verloren. Weiter wird die Abbildung durch Licht, das nach Durchdringen des Wassers oder Öls an den Hautflächen streut und zurück in das Prisma gelangt, weiter verfälscht.

### Darstellung der Erfindung

Die vorliegende Erfindung liegt der Aufgabe zugrunde, eine Vorrichtung zur Aufnahme eines optischen Kontrastbildes eines Papilarrillenmusters eines Fingers zu schaffen, bei der das Licht an der Auflagefläche für den Finger interne Totalreflexion erfährt. Insbesondere sollen die erwähnten Nachteile des Standes der Technik vermieden werden und ein hoher Kontrast des Bildes des Papilarrillenmusters unabhängig von den Eigenschaften der Fingeroberfläche, wie zum Beispiel nass oder trocken, erreicht werden.

Diese Aufgabe wird durch eine Vorrichtung gemäss dem Anspruch 1 gelöst.

Die Vorrichtung weist einen transparenten Körper auf mit einer Grenzfläche, die als Auflagefläche für die abzubildende Fingeroberfläche dient. Eine Lichtquelle ist so angeordnet, dass ihr Licht durch den transparenten Körper strahlt und auf die Grenzfläche des Körpers fällt, auf welcher der Finger liegt. Ferner weist die Vorrichtung einen Lichtdetektor und eine Abbildungsoptik auf, deren optische Achse durch eine Seitenfläche des transparenten Körpers führt. Erfindungsgemäss ist der Einfallswinkel des Lichts von der Lichtquelle auf die Grenzfläche kleiner als der kritische Winkel für interne Totalreflexion an der Grenzfläche des transparenten Körpers mit darüberliegender Luft. Die Abbildungsoptik ist so ausgelegt, dass sie nur Lichtstrahlen einfängt und auf den Lichtdetektor leitet, die von der Auflagefläche des transparenten Körpers in einem Winkel zur Normalen zur Auflagefläche ausgehen, der gleich dem oder grösser ist als der kritische Winkel für interne Totalreflexion an der Grenzfläche zwischen dem transparenten Körper mit daraufliegendem Wasser, Öl oder ähnlichem Medium.

Das Licht, das von der Lichtquelle durch den transparenten Körper auf die Grenzfläche fällt, gelangt zum grössten Teil durch die Grenzfläche und zu dem daraufliegenden Finger, da der Einfallswinkel des Lichts kleiner ist als der kritische Winkel für interne Totalreflexion an der Grenzfläche. (Nur ein kleiner Teil, ca. 4%, des von der Lichtquelle ausgesandten Lichts wird spekular reflektiert.) Das Licht wird an der Oberfläche der Papilarrillen oder im Innern des Fingers gestreut. Das im Innern des Fingers gestreute Licht wird teilweise an der Grenzfläche zwischen Finger und Luft, Wasser oder Öl intern totalreflektiert und erzeugt an der Fingeroberfläche ein evaneszentes Feld, welches in den Raum ausserhalb der Fingeroberfläche exponentiell abklingt. Die evaneszente Abklingdistanz oder 1/e-Tiefe ist der Abstand von der Fingeroberfläche, bei dem die Feldstärke des elektromagnetischen Feldes noch 1/e der Feldstärke des elektromagnetischen Feldes an der Fingeroberfläche beträgt. Die Oberflächen der Berge der Papilarrillen befinden sich nahe der Auflagefläche, wobei die Distanz zwischen ihren Oberflächen und der Auflagefläche kleiner als die evaneszente oder im Bereich der evaneszenten Abklingdistanz liegt. Die von den Bergen der Papilarrillen ausgehenden evaneszenten Felder erzeugen propagierende Wellen, die in den transparenten Körper abstrahlen. Der Eintrittswinkel der durch evaneszente Felder in der Auflagefläche erzeugten propagierenden Wellen ist gleich dem kritischen oder grösser als der kritische Winkel für interne Totalreflexion.
Die von den Tälern der Papilarrillen ausgehenden evaneszenten Felder sind zu weit von der Auflagefläche entfernt, um im transparenten Körper propagierende Wellen mit noch signifikanter Feldstärke erzeugen zu können. Da die Feldstärke exponentiell mit dem Abstand der entsprechenden Fingerpartie zur Auflagefläche abnimmt, resultiert ein hoher Kontrast zwischen den Bergen und den Tälern der Papilarrillen.

Die Abbildungsoptik ist so angeordnet, dass sie nur Licht von der Auflagefläche oder seiner Objektfläche auf den Lichtdetektor lenkt, das innerhalb des Winkelbereichs für interne Totalreflexion propagiert. In diesem Bereich propagieren Lichtstrahlen, die durch die Anregung von evaneszenten Feldern zwischen den Bergen der Rillen und der Grenzfläche entstehen. Von den Bereichen der Täler der Papilarrillen ausgehende evaneszente Lichtwellen, vermögen keine propagierenden Lichtwellen von wahrnehmbarer Feldstärke in diesem Winkelbereich zu erzeugen. Es geht also kein Licht von den Tälern aus, das in diesem Winkelbereich propagiert und vom Lichtdetektor detektiert werden könnte. Es entsteht dadurch ein Kontrastbild, in dem die Berge der Papilarrillen hell und die Täler dunkel erscheinen.

Die Erfindung erbringt den Vorteil, dass unabhängig davon, ob der Finger trocken, nass, fettig oder ölig ist, die Qualität des Kontrastbilds erhalten bleibt. Ist nämlich der Finger beispielsweise nass, befindet sich in den Tälern Wasser. Das Licht, das von der Lichtquelle in die mit Wasser gefüllten Täler der Papilarrillen gelangt, wird dort nicht gestreut. Es propagiert, wie im Fall von Luft in den Tälern, durch das Wasser zu den Oberflächen der Täler. Da diese von der Grenzfläche relativ weit entfernt sind, können wiederum von dort ausgehende evaneszente Felder keine propagierenden Lichtwellen in dem Winkelbereich für Totalreflexion bewirken. Es besteht also auch im Fall eines nassen Fingers kein Licht aus den Tälern in dem Winkelbereich, in dem die Abbildung stattfindet.
Evaneszente Felder werden im Raum zwischen Rillenbergen und der Grenzfläche angeregt, unabhängig davon, ob die Fingeroberfläche nass oder trocken ist.

Der kritische Winkel für interne Totalreflexion ist je nach Medium des transparenten Körpers sowie des daraufliegenden Mediums verschieden. Der kritische Winkel ϑₖᵣᵢₜ im Fall von Wasser am Finger und auf der Grenzfläche in den Bereichen der Täler ist grösser als im Fall von Luft über der Grenzfläche. Um das propagierende Licht innerhalb des Winkelbereichs für interne Totalreflexion jeweils für beide Fälle zu detektieren, sind die Abbildungsoptik und der Lichtdetektor so ausgelegt, dass sie Licht aus dem Winkelbereich für interne Totalreflexion im Fall von Wasser detektiert. Mit dieser Auslegung werden die propagierenden Lichtwellen sowohl im Fall von Wasser als auch im Fall von Luft auf der Grenzfläche detektiert, da der kritische Winkel im Fall von Luft kleiner ist als der kritische Winkel im Fall von Wasser. Der Winkelbereich für interne Totalreflexion im Fall von Wasser deckt sich zu einem grossen Teil mit dem Winkelbereich für interne Totalreflexion im Fall von Luft. Der kritische Winkel im Fall von Wasser beträgt 62° und im Fall von Luft 42°, wobei der Winkelbereich für interne Totalreflexion, gemessen von der Normalen zur Grenzfläche von 62° bis 90° bzw. von 42° bis 90° reicht.

In der Praxis zeigt sich, dass sich der Kontrast der Abbildung eines nassen Fingers vom Kontrast der Abbildung eines trockenen Fingers kaum unterscheidet. Obwohl der Winkelbereich für interne Totalreflexion für die beiden erwähnten Anwendungen unterschiedlich gross sind, tragen weitere Faktoren wie zum Beispiel eine unterschiedlich grosse 1/e-Tiefe dazu bei, dass ein vergleichbarer Kontrast in den Abbildungen erreicht wird.

In einer ersten Ausführung der Erfindung weist die Lichtquelle eine Linse oder mehrere Linsen auf zur Kollimation oder Quasi-Kollimation des auf die Grenzfläche gesendeten Lichts. Dadurch gelangt klar kein Licht von der Lichtquelle direkt in den Winkelbereich für interne Totalreflexion in die Abbildungsoptik und auf den Lichtdetektor.

In einer besonderen Ausführung der Erfindung weist die Lichtquelle mehrere Leuchtdioden auf, die in einem ein- oder zweidimensionalen Array angeordnet sind. Die Anordnung in einem Array bewirkt eine homogene Ausleuchtung der Grenzfläche des transparenten Körpers. Während das von einer einzelnen Leuchtdiode auf eine Fläche ausgesandte Licht häufig dunkle Ringe aufweist, wird durch die Anordnung von mehreren Leuchtdioden in einem Array eine homogene Intensitätsverteilung erreicht, indem die dunklen Ringe der einzelnen Leuchtdioden von benachbarten Leuchtdioden im Array ausgeleuchtet werden. Ferner ist dank der in den Leuchtdioden integrierten Linsen das ausgesandte Licht quasi-kollimiert oder nahezu kollimiert.

In einer weiteren Ausführung ist zwischen der Lichtquelle und dem transparenten Körper ein Diffusor angeordnet. Die Verwendung eines Diffusors bewirkt ebenfalls eine homogene Intensitätsverteilung des Lichts auf der Grenzfläche des transparenten Körpers. Es erbringt weiter den Vorteil, dass für eine gewünschte Homogenität der Intensitätsverteilung auf der Grenzfläche weniger Leuchtdioden notwendig sind.

In einer weiteren Ausführung weist die Abbildungsoptik eine einzelne Linse oder ein Linsensystem auf zur Abbildung des Papilarrillenmusters auf die Detektorfläche. Hierzu sind konventionelle Linsen sowie auch holographische Linsen verwendbar.

In einer besonderen Ausführung weist die Abbildungsoptik eine Blende auf zur Verbesserung der Tiefenschärfe des Bildes auf der Detektorfläche.

In einer weiteren Ausführung der Erfindung führt die optische Achse der Abbildungsoptik senkrecht durch die Seitenfläche des transparenten Körpers, sodass keine Lichtbrechung an der Seitenfläche entsteht, welche die Abbildung des Papilarrillenmusters verzerren könnte.

In einer bevorzugten Ausführung weist der Lichtdetektor der Vorrichtung eine zwei-dimensionalen CMOS-Kamera auf. Sie ermöglicht eine relativ einfache Weiterverarbeitung der aufgenommenen Daten, zum Beispiel für eine Bildverarbeitung zwecks Identifizierung oder Verifizierung einer Person im Rahmen eines Systems für Zutrittskontrolle oder Zugriffskontrolle zu einem Gerät.

In einer alternativen Ausführung der Erfindung weist der Lichtdetektor der Vorrichtung eine CCD-Kamera auf.

In der erfindungsgemässen Vorrichtung liegt die Auflagefläche in einem Winkel kleiner als 90° zur optischen Achse des Abbildungssystems ausgerichtet. In einer bevorzugten Ausführung der Erfindung ist in Anwendung des Scheimpflugprinzips die Detektorfläche ebenfalls in einem Winkel zur optischen Achse ausgerichtet. Die Anwendung des Scheimpflugprinzips verringert Verzerrungen der Abbildung. Die Anordnung nach diesem Prinzip verursacht jedoch eine Reduzierung der Lichtintensität auf der Detektorfläche. Um diesen Verlust an Lichtintensität zu verringern, besteht in einer besonderen Ausführung der transparente Körper aus einem hochbrechendem Material. Dies bewirkt, dass der kritische Winkel für interne Totalreflexion kleiner wird, da der Brechungsindex des dichteren Mediums grösser wird. Gegeben, dass die Seitenfläche des transparenten Körpers senkrecht zur optischen Achse der Abbildungsoptik steht, vergrössert sich der Winkel zwischen der optischen Achse und der Auflagefläche gegen 90°. Demzufolge vergrössert sich nach dem Scheimpflugprinzip auch der Winkel zwischen Detektorfläche und optischer Achse. Dadurch ist der Verlust an Lichtintensität verringert.

### Kurze Beschreibung der Zeichnungen

Es zeigen
Figur 1 eine Darstellung der Vorrichtung zur Aufnahme der Papilarrillen eines Fingers mit der Lichtquelle und Abbildungsoptik,
Figur 2 ein Beispiel einer Aufnahme der Papilarrillen eines trockenen Fingers,
Figur 3 ein Beispiel einer Aufnahme der Papilarrillen eines nassen Fingers.

### Ausführung der Erfindung

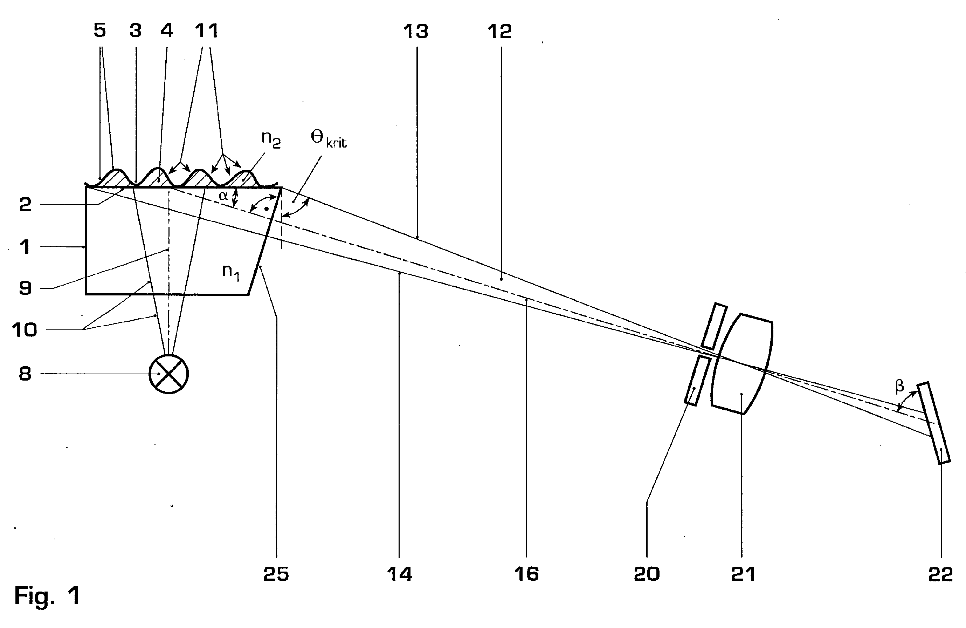

Figur 1 zeigt einen transparenten Körper 1, beispielsweise aus Glas, Quarzglas, oder Plexiglas. Seine Grenzfläche 2 dient als Auflagefläche 2 für einen Finger. Es sind hier Papilarrillen mit Bergen 3 und Tälern 4 stark vergrössert in Kontakt mit der Auflagefläche 2 gezeigt. Der Raum zwischen der Grenz- oder Auflagefläche 2 und den Oberflächen 5 der Täler 4 sind mit Luft, Wasser, Öl der auch anderen klaren Flüssigkeiten die sich am Finger einer Person befinden können, gefüllt. Die Tiefe der Täler beträgt typischerweise 1/10 mm, was viel tiefer ist als eine typische 1/e-Tiefe.
Eine Lichtquelle 8 ist unter dem transparenten Körper 1 angeordnet, wobei das austretende Licht senkrecht auf die Grenzfläche 2 gerichtet ist. Die Lichtquelle 8 besteht beispielweise aus einer oder mehreren Leuchtdioden 8, die je eine integrierte Linse aufweisen. Durch diese Linsen wird das austretende Licht grösstenteils kollimiert, wobei ein Teil des Lichts in einem Winkelbereich von beispielsweise 30° ausgesendet wird. Auf jeden Fall betragen die Einfallswinkel des Lichts auf die Grenzfläche 2 weniger als der kritische Winkel für interne Totalreflexion.
Lichtstrahlen 9 von der Lichtquelle treten durch den transparenten Körper und durch die Grenzfläche 2 auf die Oberfläche der Papilarrillen sowie in den Finger hinein, d. h. in das Innere der Berge 3 der Papilarrillen. Dort wird das Licht gestreut, und es entstehen Lichtstrahlen 10 in allen Richtungen.
Im Bereich der Berge in unmittelbarer Nähe der Grenzfläche, d.h. innerhalb der 1/e-Tiefe werden evaneszente Felder angeregt, die im transparenten Körper 1 propagierende Wellen 11 erzeugen. Diese propagieren in Winkeln (von der Normalen gemessen) die grösser /gleich dem kritischen Winkel für interne Totalreflexion sind. Konkret beträgt dieser kritische Winkel ϑₖᵣᵢₜ 62° bei einem Glasmaterial mit Brechungsindex n₁ = 1.5 und Wasser auf der Grenzfläche 2 mit n₂=1.33.
Die Abbildungsoptik ist so ausgelegt, dass nur Lichtstrahlen in dem Lichtbündel 12, die von den evaneszenten Feldern ausgehen und in einem Winkelbereich für interne Totalreflexion propagieren, detektiert werden. Die äusseren Lichtstrahlen 13 und 14 des Lichtbündels 12, welche durch die Apertur der Abbildungsoptik bestimmt werden, führen jeweils vom Rand der Auflagefläche 2 bei einem Winkel von 65° bzw. 62° aus. Sie führen durch eine Blende 20 und Linse 21 und fallen schliesslich auf den Lichtdetektor 22. Die Strahlen im Bündel 12 liegen klar innerhalb des Winkelbereichs für interne Totalreflexion an der Grenzfläche 2 mit Wasser in den Tälern 4.

Ist der Finger trocken und es befindet sich in den Tälern 4 Luft statt Wasser, so propagieren die Lichtwellen in einem Winkelbereich für interne Totalreflexion von 42° bis 90°. Davon gelangen nur die Lichtstrahlen im Winkelbereich von 62° bis 90° auf den Detektor.

Die Abbildungsoptik bewirkt eine Abbildung der Papilarrillen auf die Detektorfläche des Lichtdetektors 22 unter einer Reduktion von 4x, wobei die Auflagefläche 18 mm x 24 mm und die Detektorfläche 4mm x 6mm beträgt. In der gezeigten Ausführung besteht die Abbildungsoptik aus einer einzelnen symmetrischen Linse mit einer Brennweite von 12 mm.
In einer bevorzugten Variante ist die Abbildungsoptik mittels eines planaren Spiegels gefaltet, wodurch die Vorrichtung kompakter gestaltet werden kann. Ferner ist auch eine Faltung mittels eines gekrümmten Spiegels unter Weglassen der Linse realisierbar.

Die Gesamtsystemlänge der Abbildungsoptik beträgt im gezeigten Beispiel 80 mm. Um bei einer Objektgrösse, gemessen auf der zur optischen Achse schiefen Ebene von 24 mm eine ausreichende Tiefenschärfe zu erzielen, beträgt die Blendenapertur beispielsweise 1 mm.

Der Lichtdetektor 22 besteht aus einer zwei-dimensionalen CMOS-Kamera mit einem Array von beispielsweise 640x480 Pixeln. Mit dieser Anzahl Pixel können in dieser Anwendung Sampling- oder Quantifizierungsfehler vermieden werden.

Die optische Achse der Abbildungsoptik verläuft senkrecht durch die Seitenfläche 25 des transparenten Körpers 1. In Anwendung des Scheimpflugprinzips ist die Detektorfläche des Lichtdetektors 22 in einem Winkel α zur optischen Achse ausgerichtet, wobei die Objektfläche oder Auflagefläche in einem Winkel β zur optischen Achse liegt. In der gezeigten Ausführung betragen die Winkel α und β 23° bzw. 61°.

Um die Lichtintensität auf der Detektorfläche zu erhöhen besteht der transparente Körper aus einem hochbrechenden Material wie zum Beispiel SF-, LaF- oder LaSF-Gläser von Schott mit einem Brechungsindex zwischen 1.65 und 1.9 .
Die Verwendung solcher hochbrechenden Gläser bewirkt eine Verkleinerung des kritischen Winkels θₖᵣᵢₜ auf 37° bzw. 32°. Da die optische Achse der Abbildungsoptik senkrecht durch die Seitenfläche 25 führt, verringert sich dann auch der Winkel α zwischen Auflagefläche sowie der Winkel β zur Detektorfläche. Da durch diese Verringerung dieser Winkel eine erhöhte Lichtintensität auf der Detektorfläche erreicht wird, ist eine geringere Energie zur Beleuchtung der Grenzfläche notwendig..

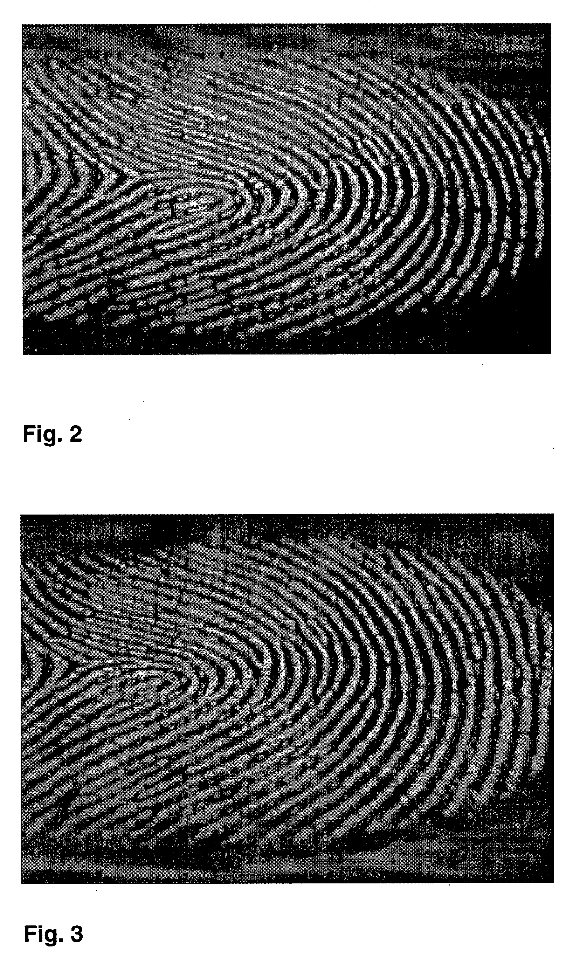

Figur 2 zeigt ein durch die erfindungsgemässe Vorrichtung erstelltes Kontrastbild der Papilarrillen eines trockenen Fingers. Die hellen Bereich des Kontrastbildes repräsentieren die Berge und die dunklen Bereiche die Täler der Papilarrillen.

Figur 3 zeigt eine durch die gleiche Vorrichtung erstelltes Kontrastbild des gleichen, jedoch nassen Fingers. Der erreichte Kontrast ist in beiden Abbildungen gleich.

### Bezugszeichenliste

- 1: transparenter Körper
- 2: Auflagefläche
- 3: Berge der Papilarrillen
- 4: Täler der Papilarrillen
- 5: Oberflächen der Täler der Papilarrillen
- 8: Lichtquelle
- 9: Optische Achse des Lichtbündels
- 10: Lichtbündel aus der Lichtquelle
- 11: Streulicht im Finger
- 12: propagierendes Lichtbündel
- 13,14: äusserste Lichtstrahlen des propagierenden Lichtbündels
- 16: optische Achse der Abbildungsoptik
- 20: Blende
- 21: Linsen
- 22: Lichtdetektor
- 25: Seitenfläche des transparenten Körpers
- θₖᵣᵢₜ: kritischer Winkel für innere Totalreflexion bei Glas-Wasser Grenzfläche
- α: Winkel zwischen Auflagefläche und optischer Achse der Abbildungsoptik
- β: Winkel zwischen Detektorfläche und optischer Achse der Abbildungsoptik
- n₁: optischer Brechungsindex des transparenten Körpers
- n₂: optischer Brechungsindex des Mediums zwischen Auflagefläche und Fingeroberfläche

## Patentansprüche

1. Vorrichtung zur optischen Aufnahme der Papilarrillen auf einer Fingeroberfläche, wobei die Vorrichtung einen transparenten Körper (1) mit einer Auflagefläche (2) für einen Finger aufweist und einer Seitenfläche (25) sowie mit einer Lichtquelle (8) zur Beleuchtung der Fingeroberfläche und einer Optik zur Abbildung der Papilarrillen, die auf der Auflagefläche (2) liegen, und mit einem Lichtdetektor (22)
**dadurch gekennzeichnet, dass**
die Lichtquelle (8) ein Lichtbündel (9) aussendet, das einen Divergenzwinkel aufweist, der kleiner ist als der kritische Winkel für interne Totalreflexion an der Auflagefläche (2) des transparenten Körpers (1), und das Lichtbündel (9) durch den transparenten Körper (1) hindurchführt und auf die Auflagefläche (2) fällt und dort die Papilarrillen des Fingers bestrahlt,
und die Optik für die Abbildung der Papilarrillen so ausgelegt ist, dass ein propagierendes Lichtbündel (12), das von der Auflagefläche (2) ausgeht und durch die Optik zur Abbildung der Papilarrillen führt und auf eine Detektorfläche des Lichtdetektors (22) fällt, in einem Winkelbereich liegt, der dem Winkelbereich entspricht, in dem Lichtstrahlen, die an der Auflagefläche (2) mit darauf liegendem Wasser durch interne Totalreflexion reflektiert werden.

2. Vorrichtung nach Anspruch 1
**dadurch gekennzeichnet, dass**
die optische Achse der Abbildungsoptik senkrecht durch die Seitenfläche (25) des transparenten Körpers (1) verläuft.

3. Vorrichtung nach Anspruch 2
**dadurch gekennzeichnet, dass**
die Auflagefläche (2) des transparenten Körpers (1) in einem ersten Winkel (α) zur optischen Achse der Abbildungsoptik und die Detektorfläche des Lichtdetektors (22) in einem zweiten Winkel (β) zur optischen Achse der Abbildungsoptik gemäss dem Scheimpflugprinzip angeordnet sind.

4. Vorrichtung nach Anspruch 3
**dadurch gekennzeichnet, dass**
die Abbildungsoptik eine Blende (20) aufweist.

5. Vorrichtung nach Anspruch 4
**dadurch gekennzeichnet, dass**
die Abbildungsoptik eine einzelne Linse oder mehrere Linsen aufweist.

6. Vorrichtung nach Anspruch 5
**dadurch gekennzeichnet, dass**
die Abbildungsoptik zwecks ihrer Faltung einen Spiegel aufweist.

7. Vorrichtung nach einem der vorangehenden Ansprüche
**dadurch gekennzeichnet, dass**
die Lichtquelle (8) mindestens eine Linse aufweist.

8. Vorrichtung nach einem der vorangehenden Ansprüche
**dadurch gekennzeichnet, dass**
die Lichtquelle (8) mindestens zwei Leuchtdioden aufweist.

9. Vorrichtung nach Anspruch 8
**dadurch gekennzeichnet, dass**
die Leuchtdioden in einem ein- oder zwei-dimensionalen Array angeordnet sind.

10. Vorrichtung nach einem der vorangehenden Ansprüche
**dadurch gekennzeichnet, dass**
zwischen der Lichtquelle (8) und dem transparenten Körper (1) ein Diffusor angeordnet ist.

11. Vorrichtung nach einem der vorangehenden Ansprüche
**dadurch gekennzeichnet, dass**
der Lichtdetektor (22) eine zwei-dimensionale CMOS-Kamera aufweist.

12. Vorrichtung nach einem der vorangehenden Ansprüche
**dadurch gekennzeichnet, dass**
der Lichtdetektor (22) eine CCD-Kamera aufweist.

13. Vorrichtung nach einem der vorangehenden Ansprüche
**dadurch gekennzeichnet, dass**
der transparente Körper (1) aus einem hochbrechenden Material besteht mit einem optischen Brechungsindex von grösser 1.55.
